# EUROPÄISCHE PATENTSCHRIFT

(11) **EP 0 623 578 B1**
(45) Veröffentlichungstag und Bekanntmachung des Hinweises auf die Patenterteilung: **13.08.1997**
(21) Anmeldenummer: 94106474.3
(22) Anmeldetag: 26.04.1994
(51) Int. Cl.: C07C 53/48, C07C 51/58

(54) **Verfahren zur Herstellung von Dichloracetylchlorid**
Process for the preparation of dichloroacetylchloride
Procédé pour la préparation du chlorure de dichloroacétyle

(30) Priorität: 01.05.1993 DE 4314381
(43) Veröffentlichungstag der Anmeldung: 09.11.1994
(73) Patentinhaber: HOECHST AKTIENGESELLSCHAFT, 65929 Frankfurt am Main (DE)
(72) Erfinder: Kampmann, Detlef, Dr., D-86368 Gersthofen (DE); Freyer, Walter, Dr., D-86391 Stadtbergen (DE); Bayer, Karl, D-86462 Langweid-Stetten (DE)

(56) Entgegenhaltungen:
- EP-A- 0 422 520
- DE-A- 2 050 562
- US-A- 4 007 224

## Beschreibung

Die Erfindung bezieht sich auf ein Verfahren zur Herstellung von Dichloracetylchlorid durch Oxidation von Trichlorethylen unter Druck und bei erhöhter Temperatur, bei dem in einem Verfahrensschritt gute Ausbeuten des Säurechlorids und niedrige Gehalte an Trichlorethylenoxid erhalten werden.

Bekanntermaßen wird Dichloracetylchlorid durch die Einwirkung von Sauerstoff oder sauerstoffhaltiger Gase, wie beispielsweise Luft, auf Trichlorethylen hergestellt. Diese Oxidation, die üblicherweise bei einer Temperatur zwischen Raumtemperatur und 200 °C und drucklos oder unter Druck durchgeführt und durch Radikalbildner oder Bestrahlung initiiert wird, führt in etwa gleichen Mengen zu Dichloracetylchlorid und Trichlorethylenoxid neben Spuren von hochsiedenden Nebenprodukten und Gasen, wie beispielsweise Chlorwasserstoff, Kohlenmonoxid und Phosgen.

Das sich als Zwischenstufe bildende Trichlorethylenoxid (Epoxid) kann durch Zugabe von stickstoffhaltigen Basen beschleunigt zum Säurechlorid umgelagert werden (vgl. US 4,007,224, US 3,884,785, US 3,509,210).

Der Nachteil der meisten bekannten Verfahren ist deren sehr lange Reaktionszeit, wobei 30 bis 90 Stunden bis zum vollständigen Umsatz die Regel sind (vgl. US 3,884,785, C.A. 109 (1988) 169876v, C.A. 101 (1984) 9093u).

Aufgrund der langen Reaktionszeit ist eine basenkatalysierte Umlagerung des Epoxides nahezu quantitativ möglich, wodurch Epoxid-Konzentrationen von < 3 % resultieren, die als unkritisch anzusehen sind.

Bekannt ist ein Verfahren zur Herstellung von Dichloracetylchlorid, bei welchem die Synthese in einem Fallfilm-UV-Reaktor unter Druck und Temperatur durchgeführt wird und erhöhte Umsatzraten erzielt werden (vgl. US 5,030,753). Die Erhöhung der Reaktionsgeschwindigkeit wird aber durch eine deutliche Steigerung der Epoxid-Konzentration im Endprodukt (ca. 7 %) begleitet. Vergleichsversuche zeigten, daß im Laufe der Reaktion sogar Epoxid-Konzentrationen von 15 - 25 % beobachtet werden können. Auch eine Erhöhung der Katalysatorkonzentration erbrachte keinerlei Verbesserung. Bedingt durch den hohen Epoxid-Gehalt ist der Umgang mit derartigen Reaktionsgemischen nicht ganz unkritisch.

Es bestand daher die Aufgabe, ein Verfahren zur Herstellung von Dichloracetylchlorid zu finden, bei welchem trotz hoher Umsatzraten niedrige Epoxid-Konzentrationen erreicht werden.

Die Aufgabe wird dadurch gelöst, daß die Synthese in Gegenwart bestimmter stickstoffhaltiger Basen durchgeführt wird.

Die Erfindung betrifft somit ein Verfahren zur Herstellung von Dichloracetylchlorid durch Umsetzung von Trichlorethylen mit Sauerstoff in flüssiger Phase und unter Bestrahlung mit kurzwelligem Licht in Gegenwart einer stickstoffhaltigen Base, dadurch gekennnzeichnet, daß die stickstoffhaltige Base ein sekundäres aliphatisches oder cycloaliphatisches Amin der Formel HNR¹R² ist, worin R¹ und R² gleich oder verschieden sind und einen Alkylrest mit 1 bis 10 C-Atomen bedeuten, wobei mindestens einer der Reste R¹ und R² eine tertiäre Struktur besitzt, oder R¹ und R² zusammen mit dem Stickstoffatom einen 5- bis 10-gliedrigen Ring, der durch eine oder mehrere Alkylgruppen substituiert sein kann, bilden, wobei mindestens eines der dem Stickstoff benachbarten C-Atome ein tertiäres C-Atom ist, und welche in einer Menge von 0,001 bis 0,1 Gew.-%, bezogen auf Trichlorethylen, eingesetzt wird.

Im erfindungsgemäßen Verfahren wird Trichlorethylen durch Oxidation in flüssiger oder gasförmiger Phase unter Bestrahlung mit kurzwelligem Licht und in Anwesenheit einer stickstoffhaltigen Base in Dichloracetylchlorid umgewandelt.

Die Reaktion wird bei einer Temperatur von 50 bis 140 °C, vorzugsweise 65 bis 120 °C und insbesondere bei 70 bis 100 °C durchgeführt. Der Druck beträgt 0,01 bis 2,0 MPa, vorzugsweise 0,03 bis 1,0 MPa und besonders bevorzugt 0,05 bis 0,5 MPa.

Als stickstoffhaltige Base wird ein sekundäres aliphatisches oder cycloaliphatisches Amin der Formel HNR¹R² eingesetzt. R¹ und R² sind gleich oder verschieden und bedeuten einen Alkylrest mit 1 bis 10 C-Atomen, vorzugsweise 3 bis 8 C-Atomen, wobei mindestens einer der Reste R¹ und R² eine tertiäre Struktur besitzen soll, oder R¹ und R² bilden zusammen mit dem Stickstoffatom einen 5-bis 10-, vorzugsweise 6- bis 8-gliedrigen Ring, der durch eine oder mehrere Alkylgruppen, vorzugsweise Methylgruppen, substituiert sein kann, wobei mindestens eines der dem Stickstoff benachbarten C-Atome ein tertiäres C-Atom ist.

Geeignete Amine sind N-Methyl-t-butylamin, N-Ethyl-t-butylamin, N-Propyl-t-butylamin, Isopropyl-t-butylamin, N-Butyl-t-butylamin, N-Isobutyl-t-butylamin, N-2-Methyl-butyl-t-butylamin, N-3-Methylbutyl-t-butylamin, N-2,3-Dimethyl-butyl-t-butylamin, N-Pentyl-t-butylamin, N-2-Ethylbutyl-t-butylamin, N-2-Ethylhexyl-t-butylamin, 2,2,6,6-Tetramethylpiperidin,2,2,6,6-Tetramethylpiperidon,4-Hydroxy-2,2,6,6-tetramethylpiperidin, bevorzugt-N-Isopropyl-t-butylamin, N-Isobutyl-t-butylamin, N-2-Methylbutyl-t-butylamin, N-2,3-Dimethyl-butyl-t-butylamin, N-2-Ethylbutyl-t-butylamin, N-2-Ethylhexyl-t-butylamin, 2,2,6,6-Tetramethylpiperidin, 2,2,6,6-Tetramethylpiperidon, 4-Hydroxy-2,2,6,6-Tetramethylpiperidin, besondersbevorzugtN-2-Methylbutyl-t-butylamin, N-3-Methylbutyl-t-butylamin, N-2,3-Dimethylbutyl-t-butylamin und 2,2,6,6-Tetramethylpiperidin.

Es hat sich gezeigt, daß sekundäre Amine mit stark abgeschirmten Stickstoffatomen auch bei erhöhtem Sauerstoffpartialdruck ihre katalytische Eigenschaft zur Umlagerung von Trichlorethylenoxid zu Dichloracetylchlorid in vollem Umfang behalten. Dies überrascht besonders, weil sekundäre Amine dieser Strukturklasse häufig als Lichtstabilisatoren für Polymere (Radikalfänger) Verwendung finden und aus diesem Grund eher einen negativen Einfluß auf die untersuchte Reaktion haben sollten.

Das Amin wird in einer Menge von 0,001 bis 0,1, vorzugsweise 0,003 bis 0,05 Gew.-%, bezogen auf eingesetztes Trichlorethylen, eingesetzt. Das entspricht einigen ppm, bezogen auf den Stickstoff. Die Menge ist abhängig von den Reaktionsbedingungen und liegt im Bereich von 1 bis 100 ppm, vorzugsweise 3 bis 50 ppm und insbesondere 5 bis 30 ppm Stickstoff.

Die Umsetzung von Trichlorethylen und Sauerstoff erfolgt in Gegenwart dieses Amins und wird durch Radikalbildner initiiert. In der bevorzugten Arbeitsweise geschieht dies durch die Bestrahlung mit kurzwelligem Licht, beispielsweise durch UV-Licht eines Hg-Hochdruckbrenners.

Der Sauerstoff wird dem Reaktionssystem kontinuierlich in einem Überschuß zugeführt, wobei ein Molverhältnis von 1,1 : 1 bis 5 : 1, vorzugsweise 1,2 : 1 bis 4 : 1 und insbesondere 1,5 : 1 bis 3,5 : 1 ausreichend ist. Nicht umgesetzter Sauerstoff wird gemeinsam mit gasförmigen Nebenprodukten kontinuierlich so entnommen, daß der Systemdruck konstant bleibt. Das Abgas wird einer basischen Abgasreinigung zugeführt, um Nebenprodukte wie beispielsweise Phosgen und HCl zu absorbieren.

Das erfindungsgemäße Verfahren läßt sich in einem Fallfilm- wie auch in einem Blasensäulenreaktor durchführen, wobei die Arbeitsweise diskontinuierlich wie auch kontinuierlich sein kann.

Im Hinblick auf eine gute Gesamtausbeute und hohe Umsatzgeschwindigkeiten wird ein Teilumsatz von 40 bis 70 % bevorzugt. Nicht umgesetztes Trichlorethylen kann problemlos destillativ abgetrennt und in das Reaktionssystem zurückgeführt werden.

### Vergleichsversuch A

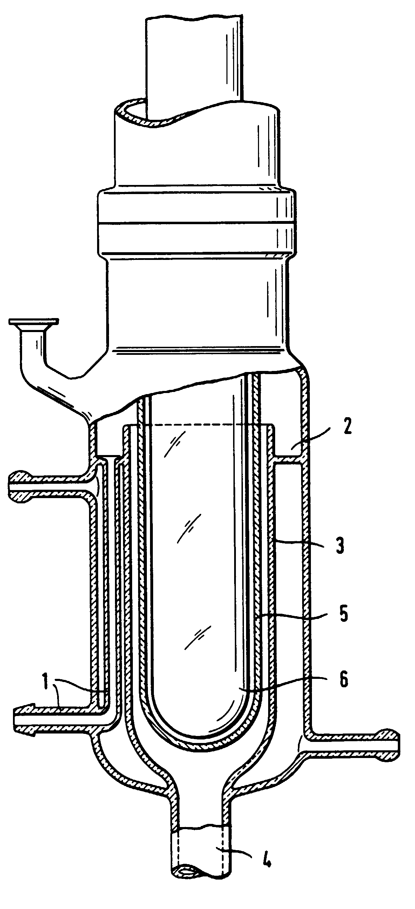

Die Versuche wurden in einem Fallfilm-Photoreaktor nach Prof. de Meijere durchgeführt. Die wichtigsten Merkmale des Reaktors sind in der Figur dargestellt. Das Reaktionsgemisch tritt durch das Rohr (1) in den Reaktor ein, füllt die Kreisrinne (2) und fließt auf der Innenseite des zylindrischen Rohres (3) als dünner Film durch den unteren Stutzen (4) zur nicht gezeigten Kreislaufpumpe.

Der Reaktor kann auch als Blasensäulenreaktor verwendet werden. In diesem Fall wird der Reaktor bis zur Kreisrinne geflutet.

In dem Schutzrohr (5) befindet sich eine UV-Lichtquelle (6) mit Kühlmantel. Derartige Photoreaktoren sind im Fachhandel erhältlich.

Der Reaktor war mit einem Vorratsgefäß (variabel von 250 cm³ bis 2000 cm³) für das Kreislaufgemisch, einem Tropftrichter für das Ausgangsmaterial und einem Intensivkühler mit nachgeschaltetem Wärmetauscher zur Abgaskühlung ausgestattet. In die Abgasleitung, die in einer NaOH-Wäsche mündete, waren ein Reduzierventil und ein Manometer integriert, die zur Einstellung des Systemdruckes benutzt wurden.

Als UV-Lichtquelle wurde ein Quecksilber-Hochdruckbrenner des Typs TQ 150 verwendet. Der Kühlmantel des Brenners wurde mit Wasser gekühlt. Der Sauerstoff wurde kontinuierlich über einen Frittenboden in den Reaktor eindosiert, die Einsatzmenge wurde mittels eines ®Rotameter bestimmt.

Für die Versuche wurde jeweils das Trichlorethylen und das Amin in die Apparatur gegeben, die Umwälzpumpe eingeschaltet und die Apparatur über einen Thermostaten aufgeheizt. Der Intensivkühler und der Wärmetauscher wurden durch einen Kryostaten temperiert (ca. - 20 °C).

Nach entsprechender Einbrennzeit des UV-Brenners wurde der Sauerstoff in konstanter Menge kontinuierlich eingeleitet. Der Abgasstrom wurde über das Reduzierventil so eingestellt, daß der vorgegebene Druck eingehalten werden konnte.

1000 g Trichlorethylen wurden mit 82 mg Pyridin (= 14,5 ppm N) versetzt und bei 75 °C in einer Blasensäulen-Fahrweise bei einem O₂-Überdruck von 0,03 MPa oxidiert. Der Gaseinsatz betrug 60 dm³/h. Die Umsetzung wurde über einen Zeitraum von 4 h beobachtet, die gaschromatografischen Analysen sind der Tabelle 1 zu entnehmen.

Der Trichlorethylen-Umsatz betrug nach 4 h 87,8 % (= 0,88 kg) zum Dichloracetylchlorid bei einer Selektivität von 77,4 %.

**Tabelle 1**

| Reaktionszeit [h] | 1 | 2 | 3 | 4 |
|---|---|---|---|---|
| Trichlorethylen [%] | 72,5 | 48,1 | 26,4 | 12,2 |
| Dichloracetylchlorid [%] | 23,1 | 40,3 | 58,0 | 68,0 |
| Trichlorethylenoxid [%] | 2,9 | 9,2 | 13,1 | 16,4 |

### Vergleichsversuch B

Wie im Vergleichsversuch A wurden 1000 g Trichlorethylen mit 82 mg Pyridin bei 75 °C umgesetzt, wobei ein O₂-Überdruck von 0,05 MPa eingestellt wurde.

Der Reaktionsverlauf ist der Tabelle 2 zu entnehmen.

Der Trichlorethylen-Umsatz betrug nach 4 h 89 % (= 0,89 kg) bei einer Selektivität von 68,5 %, bezogen auf Dichloracetylchlorid.

**Tabelle 2**

| Reaktionszeit [h] | 1 | 2 | 3 | 4 |
|---|---|---|---|---|
| Trichlorethylen [%] | 73,6 | 45,9 | 25,6 | 11,0 |
| Dichloracetylchlorid [%] | 22,2 | 37,0 | 50,7 | 61,0 |
| Trichlorethylenoxid [%] | 2,6 | 13,9 | 20,1 | 24,6 |

### Vergleichsversuch C

Wie im Vergleichsversuch B wurden 1000 g Trichlorethylen bei 75 °C und einem O₂-Überdruck von 0,05 MPa umgesetzt. Die Menge des Umlagerungskatalysators wurde verdoppelt (164 mg Pyridin).

Der Reaktionsverlauf ist in Tabelle 3 wiedergegeben.

Der Umsatz betrug nach 4 h 90,6 % (= 0,91 kg) zum Dichloracetylchlorid bei einer Selektivität von 76 %.

**Tabelle 3**

| Reaktionszeit [h] | 1 | 2 | 3 | 4 |
|---|---|---|---|---|
| Trichlorethylen [%] | 75,1 | 46,7 | 24,5 | 9,4 |
| Dichloracetylchlorid [%] | 21,9 | 42,3 | 59,0 | 68,9 |
| Trichlorethylenoxid [%] | 1,4 | 7,8 | 13,7 | 18,5 |

### Vergleichsversuch D

Wie im Vergleichsversuch C wurden 1000 g Trichlorethylen mit 164 mg Pyridin und einem O₂-Überdruck von 0,05 MPa umgesetzt. Die Reaktionstemperatur wurde auf 80 °C erhöht.

Der Trichlorethylen-Umsatz betrug nach 4 h 88,2 % (= 0,88 kg) bei einer Selektivität von 66,9 %, bezogen auf Dichloracetylchlorid.

Der Reaktionsverlauf stellte sich wie folgt dar (Tabelle 4):

**Tabelle 4**

| Reaktionszeit [h] | 1 | 2 | 3 | 4 |
|---|---|---|---|---|
| Trichlorethylen [%] | 72,3 | 47,8 | 26,1 | 11,8 |
| Dichloracetylchlorid [%] | 23,1 | 40,2 | 50,9 | 59,0 |
| Trichlorethylenoxid [%] | 2,8 | 9,5 | 20,1 | 26,0 |

### Vergleichsversuch E

Wie im Vergleichsversuch A wurden 1200 g Trichlorethylen bei 80 °C und einem O₂-Überdruck von 0,075 MPa umgesetzt. Als Umlagerungskatalysator wurde Dimethylformamid (95 mg = 15,2 ppm N) eingesetzt.

Der Reaktionsverlauf ist aus Tabelle 5 zu ersehen.

Der Trichlorethylen-Umsatz betrug nach 4 h 90,1 % (= 1,08 kg) bei einer Selektivität von 75,6 %, bezogen auf Dichloracetylchlorid.

**Tabelle 5**

| Reaktionszeit [h] | 1 | 2 | 3 | 4 |
|---|---|---|---|---|
| Trichlorethylen [%] | 60,4 | 36,8 | 20,2 | 9,9 |
| Dichloracetylchlorid [%] | 26,8 | 48,0 | 62,3 | 68,1 |
| Trichlorethylenoxid [%] | 9,0 | 12,3 | 14,3 | 19,1 |

### Vergleichsversuch F

Vergleichbar zu Versuch 5 wurden 1400 g Trichlorethylen bei 80 °C und einem O₂-Überdruck von 0,075 MPa umgesetzt. Als Umlagerungskatalysator wurde Triethanolamin (130 mg = 9 ppm N) verwendet.

Der Reaktionsverlauf ist in Tabelle 6 dargestellt.

Nach 4 h waren 81,9 % (= 1,15 kg) des Trichlorethylens bei einer Selektivität von 69,5 % umgesetzt.

**Tabelle 6**

| Reaktionszeit [h] | 1 | 2 | 3 | 4 |
|---|---|---|---|---|
| Trichlorethylen [%] | 79,7 | 57,0 | 38,2 | 18,1 |
| Dichloracetylchlorid [%] | 14,8 | 29,4 | 42,0 | 56,9 |
| Trichlorethylenoxid [%] | 4,1 | 10,2 | 16,6 | 21,8 |

### Beispiel 1

Wie im Vergleichsversuch A wurden 1200 g Trichlorethylen bei 80 °C und einem O₂-Überdruck von 0,075 MPa umgesetzt. Als Umlagerungskatalysator wurde 2,2,6,6-Tetramethylpiperidin (83 mg = 7 ppm N) eingesetzt.

Das Resultat gibt die Tabelle 7 wider.

Nach einer Reaktionszeit von 4 h waren 90,1 % (= 1,08 kg) Trichlorethylen umgesetzt. Die Selektivität, bezogen auf das Endprodukt, betrug 94,6 %.

**Tabelle 7**

| Reaktionszeit [h] | 1 | 2 | 3 | 4 |
|---|---|---|---|---|
| Trichlorethylen [%] | 66,1 | 38,8 | 20,4 | 9,9 |
| Dichloracetylchlorid [%] | 28,6 | 55,5 | 74,6 | 85,3 |
| Trichlorethylenoxid [%] | 3,4 | 3,1 | 2,2 | 1,8 |

### Beispiel 2

Wie im Beispiel 1 wurden 1400 g Trichlorethylen bei 80 °C und einem O₂-Überdruck von 0,075 MPa in einem Fallfilm umgesetzt. Es wurden 99,6 mg (= 7 ppm N) 2,2,6,6-Tetramethylpiperidin verwendet.

Der Versuchsverlauf ist in Tabelle 8 dargestellt. Es wurde ein Trichlorethylen-Umsatz von 80,32 % (= 1,12 kg), bei einer Selektivität von 93,0 %, erzielt.

**Tabelle 8**

| Reaktionszeit [h] | 1 | 2 | 3 | 4 |
|---|---|---|---|---|
| Trichlorethylen [%] | 80,0 | 60,2 | 33,4 | 19,7 |
| Dichloracetylchlorid [%] | 15,3 | 34,2 | 60,5 | 74,7 |
| Trichlorethylenoxid [%] | 3,2 | 3,1 | 3,0 | 2,4 |

### Beispiel 3

Wie im Beispiel 2 wurden 1400 g Trichlorethylen bei 80 °C und einem O₂-Überdruck von 0,075 MPa in einem Fallfilm umgesetzt. 216 mg (= 15 ppm N) 2,2,6,6-Tetramethylpiperidin wurden verwendet.

Der Reaktionsverlauf ist in Tabelle 9 aufgeführt. Der Trichlorethylen-Umsatz betrug nach 4 h 81,4 % (= 1,14 kg), wobei die Selektivität bei 95,5 % lag.

**Tabelle 9**

| Reaktionszeit [h] | 1 | 2 | 3 | 4 |
|---|---|---|---|---|
| Trichlorethylen [%] | 77,4 | 55,7 | 35,5 | 18,6 |
| Dichloracetylchlorid [%] | 20,7 | 40,4 | 61,5 | 77,8 |
| Trichlorethylenoxid [%] | 0,5 | 0,6 | 0,2 | 0,3 |

### Beispiel 4

Wie im Beispiel 3 wurden 1400 g Trichlorethylen bei 80 °C und einem O₂-Überdruck von 0,075 MPa in einem Fallfilm umgesetzt. Es wurden 96 mg (= 6 ppm N) N-2,3-Dimethylbutyl-t-butylamin als Katalysator eingesetzt.

Nach 4 h betrug der Trichlorethylen-Umsatz 82,1 % (= 1,15 kg), dabei wurde eine Selektivität von 94,7 % erzielt.

Der Reaktionsverlauf ist in Tabelle 10 dargestellt.

**Tabelle 10**

| Reaktionszeit [h] | 1 | 2 | 3 | 4 |
|---|---|---|---|---|
| Trichlorethylen [%] | 77,3 | 55,0 | 34,7 | 17,9 |
| Dichloracetylchlorid [%] | 21,0 | 42,0 | 61,7 | 77,8 |
| Trichlorethylenoxid [%] | 0,4 | 0,6 | 0,9 | 1,3 |

### Beispiel 5

Wie im Beispiel 4 wurden 1400 g Trichlorethylen bei 80 °C und einem O₂-Überdruck von 0,075 MPa in einem Fallfilm umgesetzt. Es wurden 104 mg (= 8 ppm N) N-Isobutyl-tert-butylamin als Katalysator verwendet. Nach einer Reaktionszeit von 4 h waren 82,2 % (= 1,15 kg) Trichlorethylen umgesetzt. Die Selektivität betrug 93,4 %.

Der Reaktionsverlauf ist der Tabelle 11 zu entnehmen.

**Tabelle 11**

| Reaktionszeit [h] | 1 | 2 | 3 | 4 |
|---|---|---|---|---|
| Trichlorethylen [%] | 77,5 | 56,2 | 35,9 | 17,8 |
| Dichloracetylchlorid [%] | 20,4 | 40,9 | 59,9 | 76,7 |
| Trichlorethylenoxid [%] | 0,2 | 0,5 | 1,0 | 2,0 |

### Beispiel 6

Wie im Beispiel 1 wurden 2000 g Trichlorethylen bei 80 °C und einem O₂-Überdruck von 0,075 MPa umgesetzt. Zur Umlagerung wurden 304 mg (= 15 ppm N) 2,2,6,6-Tetramethylpiperidin zugesetzt. Nach 4 h waren 68,4 % (= 1,38 kg) Trichlorethylen umgesetzt, wobei eine Selektivität von 94,1 % erzielt wurde.

Der Verlauf der Reaktion ist in Tabelle 12 dargestellt.

**Tabelle 12**

| Reaktionszeit [h] | 1 | 2 | 3 | 4 |
|---|---|---|---|---|
| Trichlorethylen [%] | 80,6 | 63,4 | 46,8 | 31,6 |
| Dichloracetylchlorid [%] | 16,1 | 33,3 | 48,5 | 64,4 |
| Trichlorethylenoxid [%] | 1,0 | 1,0 | 1,8 | 0,9 |

## Patentansprüche

1. Verfahren zur Herstellung von Dichloracetylchlorid durch Umsetzung von Trichlorethylen mit Sauerstoff in flüssiger Phase und unter Bestrahlung mit kurzwelligem Licht in Gegenwart einer stickstoffhaltigen Base, dadurch gekennnzeichnet, daß die stickstoffhaltige Base ein sekundäres aliphatisches oder cycloaliphatisches Amin der Formel HNR¹R² ist, worin R¹ und R² gleich oder verschieden sind und einen Alkylrest mit 1 bis 10 C-Atomen bedeuten, wobei mindestens einer der Reste R¹ und R² eine tertiäre Struktur besitzt, oder R¹ und R² zusammen mit dem Stickstoffatom einen 5- bis 10-gliedrigen Ring, der durch eine oder mehrere Alkyl(Methyl)gruppen substituiert sein kann, bilden, wobei mindestens eines der dem Stickstoff benachbarten C-Atome ein tertiäres C-Atom ist, und welche in einer Menge von 0,001 bis 0,1 Gew.-%, bezogen auf Trichlorethylen, eingesetzt wird.

2. Verfahren nach Anspruch 1, dadurch gekennzeichnet, daß die stickstoffhaltige Base N-Isopropyl-t-butylamin, N-Isobutyl-t-butylamin, N-2-Methylbutyl-t-butylamin, N-2,3-Dimethyl-butyl-t-butylamin, Dimethyl-butyl-t-butylamin, N-2-Ethylhexyl-t-butylamin, 2,2,6,6-Tetramethylpiperidin, 2,2,6,6-Tetramethylpiperidon oder 4-Hydroxy-2,2,6,6-Tetramethylpiperidin ist.

3. Verfahren nach Anspruch 1, dadurch gekennzeichnet, daß die stickstoffhaltige Base N-2-Methylbutyl-t-butylamin, N-3-Methylbutyl-t-butylamin, N-2,3-Dimethylbutyl-t-butylamin oder 2,2,6,6-Tetramethylpiperidin ist.

## Claims

1. A process for the preparation of dichloroacetyl chloride by reaction of trichloroethylene with oxygen in the liquid phase and with irradiation with short-wave light in the presence of a nitrogen-containing base, which comprises the nitrogen-containing base being a secondary aliphatic or cycloaliphatic amine of the formula HNR¹R² in which R¹ and R² are identical or different and are an alkyl radical having 1 to 10 carbon atoms, where at least one of the radicals R¹ and R² has a tertiary structure, or R¹ and R² together with the nitrogen atom form a 5- to 10-membered ring which can be substituted by one or more alkyl (methyl) groups, where at least one of the carbon atoms adjacent to the nitrogen is a tertiary carbon atom, the nitrogen-containing base being employed in an amount of 0.001 to 0.1% by weight based on trichloroethylene.

2. The process as claimed in claim 1, wherein the nitrogen-containing base is N-isopropyl-t-butylamine, N-isobutyl-t-butylamine, N-2-methylbutyl-t-butylamine, N-2,3-dimethylbutyl-t-butylamine, N-2-ethylbutyl-t-butylamine, N-2-ethylhexyl-t-butylamine, 2,2,6,6-tetramethylpiperidine, 2,2,6,6-tetramethylpiperidone or 4-hydroxy-2,2,6,6-tetramethylpiperidine.

3. The process as claimed in claim 1, wherein the nitrogen-containing base is N-2-methylbutyl-t-butylamine, N-3-methylbutyl-t-butylamine, N-2,3-dimethylbutyl-t-butylamine or 2,2,6,6-tetramethylpiperidine.

## Revendications

1. Procédé pour préparer du chlorure de dichloracétyle par réaction de trichloréthylène avec de l'oxygène en phase liquide, avec exposition à une lumière de courte longueur d'onde, en présence d'une base azotée, caractérisé en ce que la base azotée est une amine secondaire, aliphatique ou cycloaliphatique, de formule HNR¹R², dans laquelle R¹ et R² sont identiques ou différents et représentent chacun un groupe alkyle ayant de 1 à 10 atomes de carbone, au moins l'un des radicaux R¹ et R² ayant une structure tertiaire, ou encore R¹ et R², avec l'atome d'azote, forment un noyau ayant de 5 à 10 chaînons, lequel peut être substitué par un ou plusieurs groupes alkyle, au moins l'un des atomes de carbone voisins de l'azote étant un atome de carbone tertiaire, la base étant utilisée en une quantité de 0,001 à 0,1 % en poids par rapport au trichloréthylène.

2. Procédé selon la revendication 1, caractérisé en ce que la base azotée est la N-isopropyl-tert-butylamine, la N-isobutyl-tert-butylamine, la N-2-méthylbutyl-tert-butylamine, la N-2,3-diméthylbutyl-tert-butylamine, la N-2-éthylbutyl-tert-butylamine, la N-2-éthylhexyl-tert-butylamine, la 2,2,6,6-tétraméthylpipéridine, la 2,2,6,6-tétraméthylpipéridone ou la 4-hydroxy-2,2,6,6-tétraméthylpipéridine.

3. Procédé selon la revendication 1, caractérisé en ce que en ce que la base azotée est la N-2-méthylbutyl-tert-butylamine, la N-3-méthylbutyl-tert-butylamine, la N-2,3-diméthylbutyl-tert-butylamine ou la 2,2,6,6-tétraméthylpipéridine.
